# EUROPEAN PATENT SPECIFICATION

(11) **EP 3 770 600 B1**
(45) Date of publication and mention of the grant of the patent: **10.05.2023**
(21) Application number: 20186700.9
(22) Date of filing: 20.07.2020
(51) Int. Cl.: G01N 33/543, G01N 33/574, G01N 33/547, C12N 5/00, C12M 3/06, C12M 1/12

(54) **PURIFICATION PROCESS FOR CELLS**
REINIGUNGSVERFAHREN FÜR ZELLEN
PROCÉDÉ DE PURIFICATION DE CELLULES

(30) Priority: 25.07.2019 CH 9512019
(43) Date of publication of application: 27.01.2021
(73) Proprietor: CSEM Centre Suisse d'Electronique et de Microtechnique SA - Recherche et Développement, 2002 Neuchâtel (CH)
(72) Inventor: ZINGGELER, Marc, 4059 Basel (CH)
(74) Representative: P&TS SA (AG, Ltd.)

(56) References cited:
- EP-A1- 3 531 128
- CN-A- 105 331 516
- CN-A- 107 338 185
- US-A1- 2005 244 843
- MARC ZINGGELER ET AL: "Biophysical Insights on the Enrichment of Cancer Cells from Whole Blood by (Affinity) Filtration", SCIENTIFIC REPORTS, vol. 9, no. 1, 4 February 2019 (2019-02-04), XP055750250, DOI: 10.1038/s41598-018-37541-3
- ANNE MEUNIER ET AL: "Combination of Mechanical and Molecular Filtration for Enhanced Enrichment of Circulating Tumor Cells", ANALYTICAL CHEMISTRY, vol. 88, no. 17, 9 August 2016 (2016-08-09), pages 8510-8517, XP055750284, ISSN: 0003-2700, DOI: 10.1021/acs.analchem.6b01324
- KANGFU CHEN ET AL: "Integration of Lateral Filter Arrays with Immunoaffinity for Circulating-Tumor-Cell Isolation", ANGEWANDTE CHEMIE INTERNATIONAL EDITION, vol. 58, no. 23, 3 June 2019 (2019-06-03), pages 7606-7610, XP055750322, ISSN: 1433-7851, DOI: 10.1002/anie.201901412
- OSCAR LARA ET AL: "Enrichment of rare cancer cells through depletion of normal cells using density and flow-through, immunomagnetic cell separation", EXPERIMENTAL HEMATOLOGY, vol. 32, no. 10, 1 October 2004 (2004-10-01), pages 891-904, XP055750808, US ISSN: 0301-472X, DOI: 10.1016/j.exphem.2004.07.007
- PETRA BANKÓ ET AL: "Technologies for circulating tumor cell separation from whole blood", JOURNAL OF HEMATOLOGY & ONCOLOGY, vol. 12, no. 1, 14 May 2019 (2019-05-14), XP055750557, DOI: 10.1186/s13045-019-0735-4
- PENG LI ET AL: "Negative Enrichment of Target Cells by Microfluidic Affinity Chromatography", ANALYTICAL CHEMISTRY, vol. 83, no. 20, 15 October 2011 (2011-10-15), pages 7863-7869, XP055750566, ISSN: 0003-2700, DOI: 10.1021/ac201752s
- Qiu Xiaolong ET AL: "Microfluidic filter device with nylon mesh membranes efficiently dissociates cell aggregates and digested tissue into single cells", Lab on a Chip, vol. 18, no. 18, 1 January 2018 (2018-01-01), pages 2776-2786, XP055856771, UK ISSN: 1473-0197, DOI: 10.1039/C8LC00507A Retrieved from the Internet: URL:https://pubs.rsc.org/en/content/articl epdf/2018/lc/c8lc00507a>

## Description

### Field of the invention

The present invention concerns a process for isolating one or more populations of cells from a sample in which they are present in a low concentration.

### Description of related art

**[0001]** Identifying and isolating cells from a biological sample, such as a blood sample, is well-known for establishing a diagnostic test. Membranes are commonly used to this end, allowing to separate several populations of cells.

However, when the concentration of the cells to be identified is low compared to the other cells in the sample, the usual filtration processes can become inefficient.

In particular, the identification of circulating tumor cells (CTCs) and fetal cells can lack reliability. Due to their ultra-low concentration of only few cells per mL of blood, the cells must be isolated from a sufficiently large blood volume with high yield (i.e. close to 100 %) and sufficient purity to enable molecular diagnostic procedures using existing analytical methods, such as immunocytochemistry (ICC), polymerase chain reaction (PCR) or sequencing. Other downstream methods, such as cell culturing, xenotransplantation or drug efficacy testing, may additionally require cells with unaffected biological properties.

Targeting molecules on the surface of the target cells does not always allow to collect the corresponding cells. Furthermore, the methods currently used for this purpose, such as workflows using magnetic beads, may strongly alter the biological properties, such as viability, of the target cells. The known negative selection strategies, including workflows using magnetic beads, comprise steps such as 1) incubating the blood with magnetic beads, which are functionalized with an antibody against a blood cell surface marker and pulling them out with a magnet (e.g. using EasySepTM from STEMCELL Technologies) or 2) crosslinking WBCs with RBCs and removing them by sedimentation (e.g. using RosetteSepTM from STEMCELL Technologies). These known methods are not suitable for large blood volumes. In addition, they do not allow to isolate the target cells with sufficient purity for straight-forward analysis. They have furthermore the risk of entrapping target cells (i.e. reduced yield).

Physical separation approaches, although more suitable to retain the biological integrity of the target cells, and for high-throughput processes, lack specificity. A high yield is usually obtained with a low purity or vice versa, a high purity induces a low yield.

Affinity membranes, comprising immobilized antibodies against target cell characteristic surface proteins are known, for example from US2013255361. However, this method is strongly dependant on the expression status of the cells with regard to the selected surface markers, resulting in the loss of some important cells. Furthermore, the targeted cells are bound to the membrane surface and their retrieval often results in a lack of viability or biological functionality.

The document EP3531128 filed before the present patent application but published after, described a method based on immobilized antibodies against targeted cells. The document "biophysical insights on the enrichment of cancer cells from whole blood by affinity filtration" by Marc Zinggler et al. scientific report February 2019, relates to an affinity filtration targeting the cells of interest. The scientific articles "Enrichment of rare cancer cells through depletion of normal cells using density and flow-through, immunomagnetic cell separation" Oscar Lara et al., experimental hematology 32 (2004) October 2004, "Technology for tumor cells separation from whole blood", P. Banko et al. Journal of hematology and oncology Vol.12, N° 1, 14 May 2019, "Negative enrichment of target cells by microfluidic affinity chromatography", P. Li et al. Analytical chemistry Vol.83, N° 20, 15 October 2011, pages 7863-7869, "Combination of mechanical and molecular filtration for enhanced enrichment of circulating tumor cells", Meunier et al. Analytical chemistry Vol 88, n°17, 9 August 2016, and "Integration of lateral filter arrays with immunoaffinity for circulating-tumor-cell isolation", Kangfu Chen et al. Angewandte chemie international edition, vol. 58, N°23, 3 June 2019, provide alternative approaches to isolate circulating cells.

The documents CN 107338185, CN105331516 and US 2005244843 describe filtration processes aiming at isolating circulating cells.

The scientific publication titled "Microfluidic filter device with nylon mesh membranes efficiently dissociates cell aggregates and digested tissue into single cells", Xiaolong Qiu et al. Lab on Chip 2018, 18, 2776 provides a filtration process based on a combination of two different membranes.

There is therefore a room to improve the purification process to isolate cells being present in a biological sample in a low concentration, in particular circulating tumor cells and circulating fetal cells.

### Brief summary of the invention

It is an object of the present invention to provide a purification process which allows to isolate cells of interest in a high yield and high purity. It is also an object of the present invention to increase both the yield and the purity of a purified population of cells.

It is an object of the present invention to provide a purification process for isolating cells which are present in a mixture of cells in a ratio lower than 10⁻⁷, 10⁻⁸, or 10⁻⁹, compared to the other cell populations in the sample.

It is an object of the present invention to provide a purification process adapted to retrieve unknown cells, or cells having unknown surface markers, or cells having no linkable surface markers, or cells which cannot be immobilized on a filtration membrane.

It is a further object of the present invention to provide a purification process which does not modify the functionalities of the targeted cells, and in particular, which does not modify the surface of the targeted cells or their surface markers.

It is an object of the present invention to provide a purification process adapted for the diagnosis of pathologies or infections related to unknown cells, or cells having unknown surface markers, or cells being present in a ratio lower than 10⁻⁷, 10⁻⁸, or 10⁻⁹, compared to the other cell populations in the sample.

It is an object of the present invention to provide a diagnostic method to identify circulating tumor cells or circulating fetal cells, in particular circulating fetal trophoblastic cells, or to improve the accuracy of the diagnostic methods used to identify these circulating cells.

It is a further aim of the present invention to provide a purification process which allows to concentrate the targeted cells.

It is also an object of the present invention to provide a purification process which is easily implemented and fast.

It is a further purpose of the present invention to provide a purification device adapted to the purification of cells which are present in a biological sample in a very low concentration, or which are not known or which cannot be immobilized on a filtration membrane.

According to the invention, these aims are achieved by means of the process and the device claimed in the independent claims 1 and 11, and further detailed through the dependent claims.

Above and below, the terms "fetal cells" designate any cell of a fetus, from its early embryo stage until its birth.

### Brief Description of the Drawings

The invention will be better understood with the aid of the description of an embodiment given by way of example and illustrated by the following figures :
Fig. 1 : schematic view of the cell purification process;
Fig. 2a: diagram of cell distribution after step S1 of the purification process;
Fig. 2b: diagram of cell distribution after step S5 of the purification process;
Fig. 3 : example of crosslinking elements;
Fig. 4: schematic view of the pores of the filtration membrane;
Fig.5a : Schematic view of a purification device according to an embodiment;
Fig.5b, Fig. 5c : schematic view of the filtration device during the retrieving step S5.

### Detailed Description of possible embodiments of the Invention

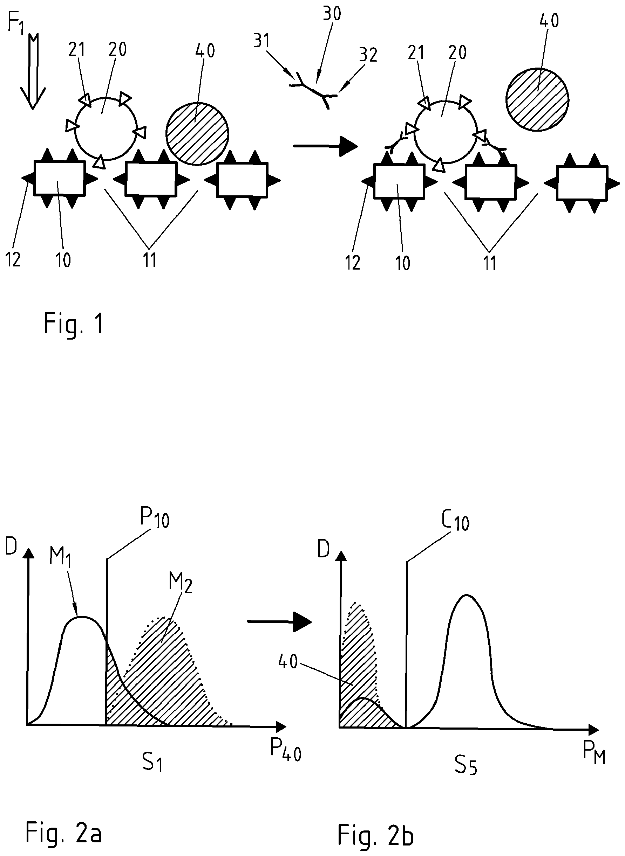

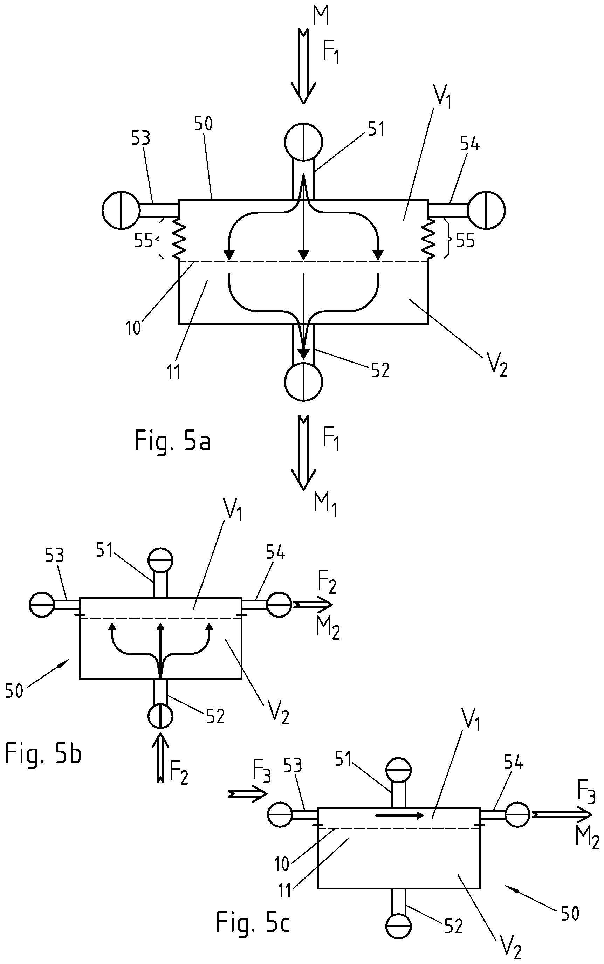

The present invention, as illustrated in figures 1-5, provides a process for isolating cells of interest **40** from a sample mixture **M** (see figure 5a) comprising unwanted or polluting cells **20.** The cells of interest **40** denote any cells which need to be identified, detected and/or analysed, for diagnostic or analytical purposes, as well as clusters thereof. A general process is illustrated in figure 1. Preferably, the cells of interest **40** should be kept alive in such a way they can be replicated in a cell culture. The cells of interests **40** should preferably also be kept functional. Their surface markers should thus be kept unmodified and substantially free of additional molecules which may disturb their biological functions. Alternatively or in addition, the cells of interest **40** may bear unknown surface markers in such a way that positive trapping using a specific linker is not possible or not suitable. Alternatively or in addition, the cells of interest **40** may be in such a low amount within the sample mixture **M** that they cannot be isolated with a sufficient yield or purity using known filtration processes. In particular, filtration processes based on physical properties of the cells may be too unspecific to isolate the cells of interest **40.** For example, the cells of interest **40** may be circulating cells within the body such as circulating tumor cells, also known as CTCs, or circulating fetal cells or other circulating cells, like infectious cells.

The cells of interest **40** may be contained in one of any biological sample including blood sample, lymphatic fluid, urine sample, or cells arising from a biopsy sample or any other biological sample, and placed in a solution within a fluid such as a physiological fluid.

The polluting cells **20** comprise all the unwanted cells present within the biological sample mixture **M.**

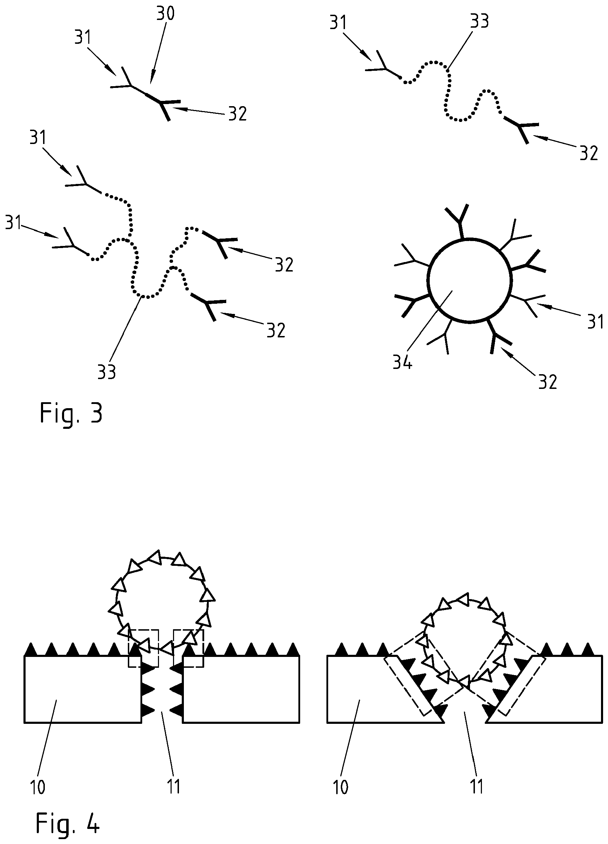

The present process comprises a filtration step **51,** consisting of filtering the sample mixture **M** of cells over a membrane **10** having pores **11.** The sample mixture **M** comprises the cells of interest **40** among a quantity of other various cells in solution. The membrane **10** denotes any mesh or filtration device allowing to separate the cells of the sample mixture **M** based on their size or stiffness or both size and stiffness or other physical cell properties (e.g. nuclei size or stiffness). The cells of interest **40** have some physical parameters **P40,** which can be used to partially isolate them from the sample mixture **M.** The physical parameters **P40** of the cells of interest **40** include the size of the cells of interest **40.** The physical parameters **P10** of the membrane **10** are preferably defined according to the physical parameters **P40** of the cells of interest **40.** The physical parameters **P10** of the membrane **10** include the number, the size and the shape of the pores **11** (see figure 4). The physical parameters **P10** of the membrane **10** allows to define a threshold to separate the retrieved mixture **M2** and the waste mixture **M1,** during the filtration step **51,** as shown in figure 2a. The cell distribution **D** within the waste mixture **M1** and the retrieved mixture **M2** may be similar, almost similar or different.

The membrane **10** may be a track-etched or a high-precision membrane. The membrane **10** may be for example selected from a polymer, a metal, a glass or a silicon based membrane like a silicon nitride material. It is to be understood that any suitable material may be used in relation to the purpose of the present process. The membrane **10** comprises pores **11.** The pores **11** of the membrane **10** have a size comprised between 4 µm to 12 µm, preferably 5 µm to 8 µm. The pore size is preferably selected according to the size of the cells of interest **40.** The pores **11** of the membrane **10** may have a cylindrical shape, or a conical shape or a slotted shape. A given membrane **10** may comprise a series of pores having a given shape and a series of pores having another shape. The membrane **10** having tapered pores **11,** as shown in figure 4 is preferred due to the increased contact area between the membrane **10** and the cells of the mixture **M,** compared to a cylindrical pore **11.** The density of pores **11** within the membrane **10** may be selected according to the needs of the filtration. For example, a density of few thousands of pores per cm² may be suitable. The density of pores **11** may be higher than this, up to 1 million per cm². The thickness of the membrane **10** may be comprised between 1 µm to 50 µm, preferably between 5 µm to 30 µm.

According to an embodiment, more than one membrane **10** may be used for the filtration step **S1.**

The membrane **10** is preferably coated with a cell repellent material in such a way to avoid unspecific adsorption on the membrane **10.** In particular the membrane **10** may be coated with a hydrophilic uncharged polymer. The coating includes known hydrogels and polymers commonly used for this purpose. Preferably, the cell repellent coating is covalently bound to the membrane **10.** The cell repellent coating is advantageously swelling when exposed to an aqueous solution. The linear swelling factor may be equal or higher than 1.5.

The membrane **10** is advantageously included or integrated to a filtration device **50** (see figures 5a-c) comprising at least one inlet **51** to supply the mixture **M** of cells to the membrane **10** and at least one outlet to collect the eluted waste mixture **M1,** comprising the cells and other material which has passed through the pores **11** of the membrane **10.** The filtration flow **F1** goes from the inlet **51** toward the outlet **52** through the membrane **10.**

The filtration step **S1** is preferably performed at room temperature, namely around 20°C. A range of temperature comprised between 15°C and 25°C is also applicable. Alternatively, a colder temperature may be used. The filtration device **50** may be thermo-regulated in such a way that the temperature during the filtration process is controlled, either for the filtration step **51,** either for another step of the filtration process.

The filtration step **51** may involve a pressure gradient or a pressure difference between the two opposite sides of the membrane **10.** An overpressure may be supplied in the direction of the filtration flow **F1** upstream the membrane **10.** Alternatively or in addition, an underpressure may be applied downstream the membrane **10.** According to a variant, a centrifugation force may be applied instead of an overpressure or an underpressure. The filtration device **50** may comprise one or more pressure sensors and a means for regulating the pressure. One or more means to induce a controlled hydrostatic pressure or pumps, such as peristaltic pumps may be connected or integrated to the filtration device **50.** The pressure may be comprised between 20 to 200 mbar, preferably between 30 and 50 mbar. Lower pressures such as around 1 mbars or 10 mbars may also be applied, especially for diluted blood sample.

The time of the filtration step **S1** is determined to avoid the degradation of the cells. It is preferably lower than one minute, typically comprised between 1 and 60 seconds. It is advantageous to have a membrane size or a density of pores or both membrane size and density of pores large enough to allow a fast filtration. A range of 200'000 to 400'000 pores per mL of the sample mixture **M** may be a suitable ratio. Other specific pore number may be considered depending on the type of sample mixture **M** to be dealt with.

Based on their physical properties, a part of the cells of the sample mixture **M** are eluted through the membrane **10,** forming a waste mixture **M1,** and another part of the cells remain on the membrane **10,** forming a retrieved mixture **M2** of trapped cells. The physical parameters **P10** of the membrane **10** and **P40** of the cells of interest **40** contribute to define the cell distribution **D** during the filtration step **S1.** In addition, the conditions under which the filtration step **S1** is performed can be modulated to increase the part of the cells of interest **40** within the retrieved mixture **M2.** In particular, the temperature and the pressure of the sample mixture **M** as well as the duration of filtration, may be monitored and controlled. The physical parameters, including the size and the shape of the pores **11** of the membrane **10,** are determined in a way that the cells of interest **40** remain within the retrieved mixture **M2.**

The process of the present invention comprises a first washing step **52.** The first washing step **52** is performed by supplying a suitable buffer in the direction of the filtration flow **F1.** The first washing step **52** may be understood as a front-washing step. Preferably, the physical conditions of the first washing step, such as the pressure gradient or pressure difference, and the temperature, are the same than those applied during the filtration step **S1.** Alternatively, one or more physical parameter may be different. The time of the first washing step **52** is also limited to one minute, preferably lower than 30 seconds, most preferably lower than 10 seconds. The suitable buffer can be supplied through the inlet **51** of the filtration device **50,** already used for supplying the mixture **M** to the membrane **10.** Alternatively, the suitable buffer can be supplied through another inlet **53.** In a preferred arrangement, the same inlet is used for the filtration step **S1** and the first washing step **S2.** Preferably, the filtration step **51** and the first washing step **S2** are used in a single continuous process. An example of such a continuous process is described in Zinggeler et al. Sci. Rep. 2019. To this end, the biological sample to be filtered may be contained in a container (not shown) allowing containing the sample mixture **M** and the buffer used for the first washing step **S2** in such a way that the buffer and the sample mixture **M** do not mix. Such a container may take the shape of a spiral tube allowing feeding the filtration device **50** with the sample mixture **M** and the buffer in a sequential and continuous way.

The first washing step **52** is preferably performed under a difference of pressure from one side of the membrane **10** to the other side. To this end, an overpressure may be applied on the side of the membrane **10** wherein the sample mixture **M** is supplied. Alternatively or in addition, an under-pressure may be applied downstream the membrane **10** to its surface opposite from the surface on which the sample mixture is supplied.

After the filtration step **S1** and the first washing step **S2** are performed, the retrieved mixture **M2** of the trapped cells comprises the cells of interest **40** and several polluting cells **20.** The type of the polluting cells **20** is preferably known. In particular one or more of the surface markers **21,** characterising the polluting cells **20** are known and can be targeted with a specific complementary binding element **31.** Alternatively, one or more subpopulations of the polluting cells **20** are known.

Preferably, the surface concentration **PM** of a given surface marker **21** is significantly higher for the polluting cells **20** than for the cells of interest **40.** In other words, a surface marker **21** which is targeted to differentiate the polluting cells **20** and the cells of interest **40** is chosen in such a way that its surface concentration is significantly higher on the surface of the polluting cells **20,** or a subpopulation of a polluting cells **20,** than on the surface of the cells of interest **40.** The surface concentration of a given surface marker **20** is significantly higher for a population of cells than for another one if it is at least twice, or ten times or hundred times higher than the surface concentration of the same surface marker **20** of another population of cells.

A binding element **31** denotes any small molecule, peptide, polypeptide, DNA oligomers or RNA oligomers, an antibody or a part of an antibody, or any other biochemical component able to specifically bind to a given surface marker **21** of the polluting cells **20.** It is to be considered that the waste mixture **M2** comprises several type of polluting cells **20,** each of those having specific surface markers **21.** Therefore, several specific binding element **31** may be used in such a way that most of the polluting cells **20** can be bond. All the binding elements **31** are selected in a way to not bind the targeted cells **40,** or at least to preferably not bind the targeted cells **40.**

The surface markers **21** related to the polluting cells **20** may be CD45, CD2, CD14, CD16, CD17, CD19, CD31, CD53, CD61, CD63, CD66b, CD69, CD81, CD84, glycophorin A, or any other suitable surface marker.

The binding elements **31** comprise or are combined with a linking element **32** able to cooperate with a linker **12** present on the surface of the membrane **10.** The combination of the binding element **31** and the linking element **32** defines a crosslinking element **30** able to chemically bind the polluting cells **20** onto the surface of the membrane **10.** The term chemically means that the polluting cells **20** are not only retained on the membrane **10** due to their physical properties but through chemical binding. The chemical binding includes weak interactions such as electrostatic or polar interactions and stronger interactions like covalent bonds. Preferably, the crosslinking elements **30** are defined to establish strong interactions between the linker **12** of the membrane **10** and the surface markers **21** of the polluting cells **20.**

Figure 3 shows various embodiments concerning the crosslinking element **30.** The crosslinking element **30** may comprise a spacer **33** between the binding element **31** and the linking element **32.** A spacer may be or may comprise any component able to increase the distance between the binding element **31** and the linking element **32** to facilitate the binding of the polluting cells **20.** The spacer thus includes any molecular component, such as oligomers, organic polymers, micro-particles, or nano-particles **34.** The spacer **33** may allow to combine one type of binding element **31** with a given linking element **32.** Alternatively, the spacer **33** may allow to combine two different types of binding elements **21, 21'** with a given linking element **32** or several linking elements **32, 32'.** The spacer **33** can be linear or branched. The binding elements **31** a spacer bears may be specific to the surface markers **21, 21'** of one type of polluting cells **20.** Alternatively, the binding elements **31** of a given crosslinking element **30** may be specific to the surface markers **21, 21'** of different type of polluting cells **20.** The spacer **33** may be selected among one or more of an organic polymer or a biopolymer, a peptide, a DNA or RNA oligomer, a micro-particle, and a nanoparticle.

The process of the present invention comprises a crosslinking step **S3** allowing the polluting cells **20** to be strongly bond to the surface of the membrane **10.** During this crosslinking step **S3**, the cells of interest **40** remain free, meaning unbound. The crosslinking step **S3** comprises a step **S3a** of adding a crosslinking element **30** to the retrieved mixture **M2** retained on the membrane **10.** The crosslinking element **30** may be added as a solution within a buffer or a physiological fluid. The parameters such as the pH, the concentration and the type of the crosslinking element **30** may be adapted according to the specific needs. The crosslinking element **30** may be injected to the filtration device **50** through the inlet **51** or another inlet **53.** Alternatively, a solution comprising the crosslinking element **30** may be placed in the same container as the one used for injecting the sample mixture **M** and the washing buffer in steps **S1** and **S2** into the filtration device **50,** either in separated steps or in one combined steps. According to this one step procedure, the filtration step **S1,** the first washing step **S2** and the step **S3a** of adding the crosslinking element **30** into the filtration device **50** may be performed in one continuous process.

Alternatively, the crosslinking element **30** may be stored as a dry reagent or a dry combination of reagents, such as a powder or a gel, or any other non-solubilised form, in a separate container, and added to the retrieved mixture **M2** or to an intermediate reservoir comprising a suitable solvent. A mixing step can be included to facilitate the solubilisation of the crosslinking element **30** or its homogeneity.

The crosslinking step **53** comprises an incubation step **S3b,** allowing to perform the chemical binding of the crosslinking element **30** to the membrane and to the polluting cells **20.** The incubating steps **53b** may be performed according to well-known procedures. In particular, the binding of the binding element **31** and the corresponding surface marker **21** of the polluting cells **20** may be performed under conditions suitable for preserving the cells of the retrieved mixture **M2** alive. The temperature, the pH, the duration and any other parameters are thus adaptable to the specific situation. For example, the temperature may be comprised between 18°C and 22°C, preferably around 20°C, such as a room temperature. Alternatively, the temperature may be slightly above the room temperature to speed up the bonding process. Temperatures up to around 38°C may be used. A temperature ranging from about 23°C to about 37°C is thus applicable to the present process. The incubation step **53b** lasts preferably lower than an hour, most preferably lower than 30 minutes. The crosslinking conditions preferably allows to simultaneously bind the crosslinking element **30** to the linkers **12** of the membrane **10** and to the surface markers of the polluting cells **20.**

The linker **12** on the membrane may be selected among streptavidin, biotin, a DNA or RNA oligomer, an antibody or an antibody segment, Ni-NTA complex, His-tag, an reactive agent such as an azide, an alkyne, or any other reactive chemical group.

The crosslinking step **53** may be followed by a second washing step **54** to elute the remaining reagents and unreacted elements. The second washing step **54** may be made under the same conditions as the first washing step **S2.** A suitable buffer or physiological fluid can be flowed in the direction of the filtration F1, following the front-washing procedure. The duration of the second step **53** is preferably less than one minute, such as 30 seconds or less than **10** seconds. The second washing step **54** is preferably performed under a difference of pressure from one side of the membrane **10** to the other side. To this end, an overpressure may be supplied in the direction of the filtration flow **F1** upstream the membrane **10.** Alternatively or in addition, an under-pressure may be applied downstream the membrane **10.**

The second washing step **S4** may alternatively be performed under different conditions than those of the first washing steps **S2.**

Following this second washing step **S4,** the polluting cells **20** present in the retrieved mixture **M2** are bond to the membrane **10,** whereas the cells of interest **40** remain unbound. In such a way to collect the cells of interest **40,** a retrieving step **S5** is performed to isolate the cells of interest **40** from the retrieved mixture **M2.** The retrieving step **S5** is preferably performed by washing the membrane **10** in a direction different from the filtration direction **F1.**

A reverse flow **F2** of an eluting fluid can be applied, according to a back-washing procedure. The eluting fluid is preferably a suitable buffer or a physiological fluid. A reversed difference of pressure compared to the one used during the filtration step **S1** can be applied. The flow thus enters to filtration device **50** by the outlet **52** and goes through the membrane **10** in such a way that the unbound cells are removed from the membrane **10** and brought within the flow. The flow of eluting fluid can be directed through a specific outlet **54,** allowing to collect the purified cells of interest **40.** Alternatively, the flow of eluting fluid can enter the filtration device **50** through a specific inlet allowing it to cross the membrane in a direction opposite to the filtration flow **F1.**

Alternatively, the membrane **10** can be reversed, and the flow of eluting fluid can be inject in the direction of filtration **F1.** The unbound cells are thus retrieved from the membrane **10** through the outlet **52.**

Alternatively, a laminar flow **F3** of an eluting fluid can be flowed at the surface of the membrane **10** without crossing it, according to a shear-washing procedure. For example, the eluting fluid can be injected to the filtration device **50** by a specific inlet **53,** on the side of the membrane containing the retrieved mixture **M2,** and collected through an output **54,** arranged on the same side of the membrane as the inlet **53.** The purified cells of interest **40** are thus collected.

The retrieving step **S5** may include a sequential washing procedure comprising the back-washing and the shear-washing steps or a non-sequential combination thereof, wherein two different flows **F2** and **F3** are combined.

The retrieving step **S5** can include a shaking step **S5a** wherein the membrane **10** is physically shaken in order to facilitate the separation of the cells of interest **40** from the membrane **10.** The membrane **10** can be vibrated or shaken at a frequency which is not prejudicial to the cell integrity. The shaking of the membrane **10** can be performed with or without deformation of the membrane **10.** Any suitable physical means of shaking the membrane can be used. A vibrating or shaking device may be incorporated within the filtration device **50** for this purpose. Preferably, the vibrating or shaking means exclude the use of ultrasounds, which may be prejudicial to the cell integrity. The vibration may be produced using a vortex generator for example or any equivalent known device. The shaking may be provided by the means of a translational periodical move either along one single direction or within a plan, using for example an eccentric rotational device. The shaking has low frequency compared to the vibrating. The vibrating or shaking device may allow the possibility to vary the frequency of move from a low shaking frequency, such as around 1 to 10 Hertz, to a higher vibrating frequency up to 100 or 1000 hertz, either progressively or using preselection ranges. The frequency may be higher than 1000 Hertz, provided that it remains lower that 10 000 Hertz. Alternatively, two distinct devices may be incorporated in the filtration device **50,** a first one dedicated to high frequencies for vibrations, the other one being dedicated to lower frequencies adapted for a gentle shaking. The shaking step **S5a** results in the releasing of unbound cells from the membrane.

The yield of the purification process, and in particular of the retrieving step **S5,** depends on the surface concentration **PM20** of the surface markers **20** compared to the surface concentration **PM40** of the same surface marker on the surface of the cells of interest **40.** Preferably, the concentration of the surface marker **PM20** of the polluting cells **20** or a subpopulation of polluting cells **20,** is higher than the concentration **PM40** of the same surface marker on the cells of interest **40** by a factor of 2, or 10, preferably 100, 1000 or more. Preferably, the selected surface marker **20** is absent from the cells of interest **40.** Figure 2b shows the cell distribution **D** of the cells of interest **40** and the polluting cells **20** or a subpopulation of polluting cells **20,** after the retrieving step **S5.** The chemical properties **C30** of the crosslinking element **30** includes its selectivity toward the surface marker **20** of different cells of a mixture. The chemical properties **C30** of the crosslinking element **30** allows to define a threshold to separate the cells of interest **40** and the polluting cells **20,** or a subpopulation of a polluting cells **20,** during the retrieving step **S5.**

The purification process of the present invention advantageously comprises a marker selection step **SM,** wherein the binding element **31** of the crosslinking element **30** is selected to specifically bind to a surface marker **21,** the concentration of which is significantly higher on the surface of the polluting cells **20** than on the surface of the cells of interest **40.** Several binding element **31** can be selected, each of those being selective of surface markers **21, 21'** of different subpopulations of polluting cells **20.**

In order to concentrate the purified cells of interest **40** into a volume of fluid as small as possible, the filtration device **50** may comprise a dead-volume reducing means **55.** This is preferred for many analytical or other procedures performed with the target cells. To this end, the filtration device **50** may comprise one or more flexible or adjustable walls, or part of walls, allowing to decrease the volume available on the membrane side comprising the retrieved mixture **M2.** Any other dead-volume reducing means can be used. For example, the membrane **10** can be arranged on a membrane holder which is movable in such a way to reduce the available volume on one side of the membrane **10.** The filtration process can thus comprise a reducing step **SR**, wherein the dead-volume of the device **50** is reduced, either manually, either automatically, before initiating the retrieving step **S5.**

The filtration process comprises a collecting step **S6,** wherein the purified cells of interest **40** are collected. The collected cells of interest **40** are sufficiently pure and concentrated to be analysed, identified or cultured for multiplication. The collection of the purified cells of interest **40** is preferably done at a collecting outlet **54** different from the filtration outlet **52.**

In case the collected cells of interest **40** are still polluted with one or more subtype of polluting cells **20,** the purification process can be iterated once or more times, using either the same type of membrane **10,** or a different type of membrane **10,** having different physical parameters **P10,** and using a different crosslinking element **30,** wherein the binding element **31** is selected to specifically bind to the remaining population of polluting cells **20.** In particular, the present process optionally comprises the step of reiterating once or more times the retrieving step **S5** or both filtration step **S1** and retrieving step **S5,** using a membrane **10** having either the same physical properties **P10** or different physical properties **P10** and using a crosslinking element **30** having different chemical properties **C30.**

The present invention also comprises a filtration device **50,** containing a filtration membrane **10,** wherein the filtration membrane **10** defines a first volume **V1** and a second volume **V2** within the filtration device **50.** The first volume **V1** is in fluidic communication with at least one filtration inlet **51,** adapted to inject a biological sample in contact to the membrane **10.** The filtration inlet **51** may be connected to a container, wherein the biological sample, a washing fluid or any other fluids may be stored. The second volume **V2** is in fluidic communication with at least one filtration output, adapted to collect the products eluted through the membrane **10.**

One or more means to induce a controlled hydrostatic pressure or pumps, such as pumps, such as peristaltic pumps can be connected or integrated to the filtration device **50** in such a way to provide a pressure difference between the first **V1** and the second **V2** volumes. The pressure can be higher in the first volume **V1** than in the second volume **V2.** The filtration device **50** can comprise a mean to reverse to pressure difference between the first volume **V1** and the second volume **V2,** in a way that the pressure is lower in the first volume **V1** than in the second volume **V2.**

The filtration device **50** advantageously comprises a dead-volume reducing means **55,** in a way to reduce the first volume **V1.** The dead-volume reducing mean can comprise an adjusting wall or part of wall, allowing to reduce the internal volume of the filtration device **50,** in particular to reduce the internal first volume **V1** defined by the membrane **10.**

The first volume **V1** is advantageously in fluidic communication with a second inlet **53** and a second outlet **54,** allowing to flow an eluting fluid along the membrane **10.**

The filtration device **50** is conceived in such a way that it comprises at least two outlets, one of the two outlet being in fluidic connexion with the first volume **V1** and the other outlet being in fluidic connexion with the second volume **V2.**

Optionally, the membrane **10** can be reversed in such a way to allow a back-washing procedure without inversing the pressure difference.

The filtration device **50** can in addition comprise a mixing chamber (not represented) allowing to introduce one or several components in a solid or semi-solid form into a solvent, and to solubilise such components into the solvent. Such a mixing chamber is preferably in fluidic connexion with the first volume **V1.**

The filtration device **50** may in addition comprise one or more than one vibrating or shaking arrangement adapted to shake or vibrate the membrane **10.**

Any one of the vibrating or shaking arrangement and pressure means can be independently activated, either manually or automatically. In a preferred arrangement, the filtration device **50** according to the present invention comprises a control unit (not represented) allowing to automatically pilot the filtration device **50** according to one or more preselected programs. The control unit allows in particular to induce and stop the steps of the purification process above-described according to a given program. The control unit may store several predefined programs. It may alternatively or in addition allow the user to set up new programs, corresponding to specific step arrangement of the present process, by means of one or more human machine interface such as a display, a keyboard, or any other known command devices.

The filtration device **50** may in addition comprise one or more sensors, such as pressure sensors, temperature sensors, optical sensors. It may be provided for example with a sensor adapted for counting particles in a flow of fluid.

The filtration device **50** may in addition comprise alert means such as sound generator or visual alarm means, so as to alert the user in case one or more parameters is sensed as being erroneous or different from a predefined value, or outside a predefined range of values. Over pressures or too high temperatures, or unexpected fluctuation of a parameter may thus be recorded and trigger an alarm message.

In another embodiment of the filtration device, the membrane **10** is contained in an insert which is compatible with centrifugation tubes. The process will then be run by a defined sequence of centrifugation steps which are executed manually or by a robot. The filtration pressure will be controlled by the rotation speed (g-force). To perform the retrieving step **S5** the filter insert can be flipped around. To this end, the filtration device **50** may be provided with the necessary centrifugation means.

In another embodiment of the filtration device, the membrane **10** is contained in a tip which is compatible with pipettes or pipetting robots. The process will then be run by a defined sequence of pipetting steps which are executed manually or by a robot. To this end, the filtration device **50** may be provided with the necessary pipetting means.

The present invention further covers a diagnostic process comprising the purification process described above. In particular, the diagnostic process comprises a first step of retrieving a sample mixture **M** from the body. The sample mixture may be a sample of blood or a sample of urine or a sample of other body fluid or tissue. The diagnostic process further comprises a purification step by the means of the above-described purification process, in such a way that the targeted cells are isolated in a viable form. The diagnostic process may include a step of multiplying the isolated cells and analysing them. The diagnostic process is particularly adapted for early stage cancers or early stage metastatic phase identification, in particular because it is based on cells, such as CTCs cells, which are present at very low concentration. This furthermore allows to gain relevant information related to the therapy to be applied. The present diagnostic process is also advantageously applicable to prenatal diagnostic operations. In particular, fetal circulating cells may be involved in the present process to diagnose diseases before the birth. Circulating fetal trophoblastic (CFTCs) cells may be of particular interest for the purpose of the present non-invasive diagnostic test. Such a diagnostic test may be applied for the diagnosis of genetic abnormalities. For example, the Down Syndrome may be diagnosed according to the present diagnostic process.

The present diagnostic process may be applied in the same way as described here to cells other than CTCs cells or circulating fetal cells if these targeted cells are in very low concentration or have unknown surface markers. The present method is especially well suited for large sample volumes (large standard tube with 7.5 ml or 2 to 14 tubes of this volume) and low target cell concentrations (less than 100 cells/ml or less than 10 cells/ml or less than 1 cell/ml).

### Example 1: (manual CTC-isolation by hydrostatic pressure filtration)

Used membrane : commercially available track-etched membrane disc with 25 mm diameter and pore size 8 µm (e.g. track-etched polycarbonate PCT8025100 from Sterlitech Corp.) or custom-made high-precision membrane disc with 25 mm diameter, 7.2 µm cylindrical pores arranged in hexagonal packaging and porosity of 23 % and thickness of 15 µm made by hot-embossing in polycarbonate or by electroplating of nickel
The membrane is coated with hydrophilic polymer and functionalized with streptavidin linker by the process described in Zinggeler et al. Sci. Rep. 2019
The membrane is inserted in filter capsule (e.g. SX0002500 from Sigma-Aldrich). The Filter capsule is filled and washed with buffers (1. PBS-0.1% tween20, 2. PBS) using a syringe.
Undiluted EDTA blood sample (2 ml for track-etched membrane or 7.5 ml for high-precision membrane) is filtered at 34 mbar hydrostatic pressure and washed at the same pressure with PBS in one continuous step within 40 seconds using a setup requiring manual handling as described in Zinggeler et al. Sci. Rep. 2019.
Biotinylated anti-CD45 antibody solution (BAM1430, R&D Systems corp., diluted to 2 µg/ml in PBS) is injected into the capsule using a syringe and incubated at room temperature for 15 min.
Target cells are collected in a standard centrifugation tube by backwashing at 6 mbar hydrostatic pressure for 30 seconds.

### Numbers and reference signs of the figures

- 10: Membrane
- 11: Pores
- 12: Linker
- 20: Polluting cells
- 21, 21': Surface marker
- 30: Crosslinking element
- 31: Binding element
- 32, 32': Linking element
- 33: Spacer
- 34: Nanoparticle/micro-particle
- 40: Cells of interest
- 50: Filtration device
- 51, 53: Inlet
- 52: outlet
- 54: Collecting outlet
- 55: Dead-volume reducing means
- C30: Chemical properties of the crosslinking element 30
- D: Cell distribution
- F1: Filtration flow
- F2: Reversed flow
- M: Sample mixture
- M1: Waste mixture
- M2: Retrieved mixture
- P10: Physical properties of the membrane 10
- P40: Physical properties of the cells of interest 40
- PM: Surface concentration of a surface marker
- PM20: Surface concentration of a given surface marker at the surface of the polluting cells 20
- PM40: Surface concentration of a given surface marker at the surface of the cells of interest 40
- S1: Filtration step
- S2: First washing step
- S3: Crosslinking step
- S3b: Incubation step
- S4: Second washing step
- S5: Retrieving step
- S5a: Shaking step
- S6: Collecting step
- SM: Marker selection step
- SR: Reducing step
- V1: First volume
- V2: Second volume

## Claims

1. A process for isolating cells of interest (40) from a sample mixture (M) comprising polluting cells (20) comprising one or more known surface markers (21), the process comprising a filtration step (S1) through a membrane (10), wherein the sample mixture (M) is passing through a membrane (10) following a filtration flow (F1), wherein the membrane (10) is provided with pores (11) and with linkers (12), **characterized in that** the process comprises a crosslinking step (S3) allowing to bind the polluting cells (20) to the membrane (10) by the mean of a crosslinking element (30) and to maintain the cells of interest unbound (40) and a retrieving step (S5), allowing to collect the cells of interest (40), wherein said crosslinking element (30) comprises at least one binding element (31) able to specifically bind to said one or more surface marker (21) of the polluting cells (20) or a subpopulation of polluting cells (20), and at least one linking element (32) adapted to cooperate with the linkers (12) of the membrane (10) and wherein
the filtration step (S1) provides a waste mixture (M1) eluted through the membrane (10) and a retrieved mixture (M2) remaining within the membrane (10), and **in that** the size and the shape of the pores (11) are determined in a way to maintain the cells of interest (40) within the retrieved mixture (M2).

2. Process according to claim 1 , **characterized in that** the cells of interest (40) are present in said sample mixture (M) in a ratio lower than 10 ⁷, 10⁻⁸ or 10⁻⁹, compared to the other cell populations in the sample.

3. Process according to any one of the preceding claims, **characterized in that** the crosslinking step (S3) comprises a step (S3a) of placing a crosslinking element (30) in contact to the polluting cells (20) and the membrane (10), and wherein the crosslinking step (S3) comprises an incubation step (S3b).

4. Process according to one of claims 1 to 3, **characterized in that** the crosslinking element (30) further comprises a spacer (33) between the binding element (31) and the linking element (32).

5. Process according to one of claims 1 to 4, **characterized in that** during said retrieving step (S5) a flow (F2) of an eluting fluid is applied through the membrane (10) in a direction opposite to the filtration flow (F1) or a flow (F3) is applied along the membrane (10) on its side containing the cells of interest (40).

6. Process according to claim 5, the retrieving step (S5) comprising a controlled shaking step (S5a) facilitating the releasing of unbound cells from the membrane (10).

7. Process according to any one of the preceding claims, **characterized in that** the cells of interest (40) are circulating tumor cells, fetal cells, or infectious cells.

8. Process according to any one of the preceding claims, **characterized in that** it further comprises a marker selection step (SM), wherein the binding element (31) of the crosslinking element (30) is selected to specifically bind to a surface marker (20), the surface concentration (PM20) of which is significantly higher at the surface of the polluting cells (20) or a subpopulation of polluting cells (20), compared to its surface concentration (PM40) at the surface of the cells of interest (40).

9. Process according to one of claims 1 to 8, wherein said sample mixture (M) is selected among body fluids such as a blood sample, lymphatic fluid, urine sample, a biopsy sample.

10. Process according to one of claims 1 to 9, wherein the concentration of the surface marker (PM20) of the polluting cells (20) or a subpopulation of the polluting cells (20) is higher than the concentration (PM40) of the same surface marker on the cells of interest (40) by a factor 2, or 10 or 100 or 1000 or higher, or the selected surface marker of the polluting cells (20) is absent from the cells of interest (40).

11. A filtration device (50) comprising :
- a porous membrane (10) as defined in claim 1, comprising linkers (12) on its surface able to chemically bind crosslinking element (30) able to chemically bind polluting cells (20) onto its surface and to maintain the cells of interest unbound (40), said membrane defining a first volume (V1) and a second volume (V2), wherein
- the first volume (V1) is in fluidic connexion with an inlet adapted to inject a sample mixture (M) in contact to the membrane and the second volume (V2) is in fluidic connexion with an outlet adapted to collect the products eluted through the membrane (10),
- one or more pumping means adapted to provide a pressure difference between the first volume (V1) and the second volume (V2) so that pressure is higher in the first volume (V1) than in the second volume (V2),
**characterized in that** the filtration device (50) further comprises a means to reverse the pressure difference in a way that the pressure becomes lower in the first volume (V1) than in the second volume (V2) and a retrieving outlet (54) in fluidic connexion with the first volume (V1) from which the cells of interest can be retrieved, or a specific inlet (53) on the side of the membrane adapted to maintain the cells of interest and an output (54) arranged on the same side of the membrane than the inlet (53) from which the cells of interest can be retrieved.

12. Filtration device (50) according to claim 11, **characterized in that** it comprises a dead-volume reducing mean (55) allowing to reduce the first volume (V1).

13. Filtration device according to claims 11 or 12, further comprising a shaking or vibrating arrangement to shake or vibrate the membrane (10) at a frequency lower than 10 000 hertz.

14. A diagnostic process for identifying and detecting cancer, early phase cancer, genetic abnormalities such as Down syndrome **characterized in that** it comprises a purification process as described in claims 1 to 10, using a filtration device according to claims 11 to 13.

## Patentansprüche

1. Verfahren zur Isolierung von Zellen von Interesse (40) aus einem Probengemisch (M), das verunreinigende Zellen (20) umfasst, die einen oder mehrere bekannte Oberflächenmarker (21) aufweisen, wobei das Verfahren einen Filtrationsschritt (S1) durch eine Membran (10) umfasst, wobei das Probengemisch (M) durch eine Membran (10) nach einem Filtrationsfluss (F1) hindurchgeht, wobei die Membran (10) mit Poren (11) und mit Linkern (12) versehen ist, **dadurch gekennzeichnet, dass** das Verfahren einen Vernetzungsschritt (S3) umfasst, der es ermöglicht, die verunreinigenden Zellen (20) mittels eines Vernetzungselements (30) an die Membran (10) zu binden und die Zellen von Interesse ungebunden (40) zu halten, und einen Rückgewinnungsschritt (S5), der es ermöglicht, die Zellen von Interesse (40) zu sammeln, wobei das Vernetzungselement (30) mindestens ein Bindungselement (31) umfasst, das ausgebildet ist, spezifisch an einen oder mehrere Oberflächenmarker (21) der verunreinigenden Zellen (20) oder einer Subpopulation von verunreinigenden Zellen (20) zu binden, und mindestens ein Verknüpfungselement (32) umfasst, welches ausgebildet ist, um mit den Linkern (12) der Membran (10) zusammenzuwirken, und wobei
der Filtrationsschritt (S1) ein Abfallgemisch (M1), das durch die Membran (10) eluiert wird, und ein zurückgewonnenes Gemisch (M2), das in der Membran (10) verbleibt, bereitstellt, und dass die Größe und die Form der Poren (11) so bestimmt sind, dass die Zellen von Interesse (40) in dem zurückgewonnenen Gemisch (M2) erhalten bleiben.

2. Verfahren nach Anspruch 1, **dadurch gekennzeichnet, dass** die Zellen von Interesse (40) in dem Probengemisch (M) in einem Verhältnis von weniger als 10⁻⁷ , 10⁻⁸ oder 10⁻⁹ im Vergleich zu den anderen Zellpopulationen in der Probe vorhanden sind.

3. Verfahren nach einem der vorhergehenden Ansprüche, **dadurch gekennzeichnet, dass** der Vernetzungsschritt (S3) einen Schritt (S3a) umfasst, bei dem ein Vernetzungselement (30) in Kontakt mit den verunreinigenden Zellen (20) und der Membran (10) gebracht wird, und wobei der Vernetzungsschritt (S3) einen Inkubationsschritt (S3b) umfasst.

4. Verfahren nach einem der Ansprüche 1 bis 3, **dadurch gekennzeichnet, dass** das Vernetzungselement (30) ferner einen Abstandshalter (33) zwischen dem Bindungselement (31) und dem Verknüpfungselement (32) umfasst.

5. Verfahren nach einem der Ansprüche 1 bis 4, **dadurch gekennzeichnet, dass** während des Rückgewinnungsschritts (S5) ein Fluss (F2) eines eluierenden Fluids durch die Membran (10) in einer dem Filtrationsfluss (F1) entgegengesetzten Richtung angelegt wird oder ein Fluss (F3) entlang der Membran (10) auf ihrer die Zellen von Interesse (40) enthaltenden Seite angelegt wird.

6. Verfahren nach Anspruch 5, wobei der Rückgewinnungsschritt (S5) einen kontrollierten Schüttelschritt (S5a) umfasst, der die Freisetzung von ungebundenen Zellen von der Membran (10) erleichtert.

7. Verfahren nach einem der vorhergehenden Ansprüche, **dadurch gekennzeichnet, dass** die Zellen von Interesse (40) zirkulierende Tumorzellen, fötale Zellen oder infektiöse Zellen sind.

8. Verfahren nach einem der vorhergehenden Ansprüche, **dadurch gekennzeichnet, dass** es ferner einen Markerauswahlschritt (SM) umfasst, bei dem das Bindungselement (31) des Vernetzungselements (30) so ausgewählt wird, dass es spezifisch an einen Oberflächenmarker (20) bindet, dessen Oberflächenkonzentration (PM20) an der Oberfläche der verunreinigenden Zellen (20) oder einer Teilpopulation von verunreinigenden Zellen (20) im Vergleich zu seiner Oberflächenkonzentration (PM40) an der Oberfläche der Zellen von Interesse (40) deutlich höher ist.

9. Verfahren nach einem der Ansprüche 1 bis 8, wobei das Probengemisch (M) aus Körperflüssigkeiten wie einer Blutprobe, einer Lymphflüssigkeit, einer Urinprobe oder einer Biopsieprobe ausgewählt ist.

10. Verfahren nach einem der Ansprüche 1 bis 9, wobei die Konzentration des Oberflächenmarkers (PM20) der verunreinigenden Zellen (20) oder einer Subpopulation der verunreinigenden Zellen (20) um einen Faktor 2 oder 10 oder 100 oder 1000 oder höher ist als die Oberflächenkonzentration (PM40) desselben Oberflächenmarkers auf den Zellen von Interesse (40), oder der ausgewählte Oberflächenmarker der verunreinigenden Zellen (20) auf den Zellen von Interesse (40) fehlt.

11. Eine Filtrationsvorrichtung (50), umfassend:
- eine poröse Membran (10) nach Anspruch 1, die auf ihrer Oberfläche Linker (12) aufweist, die ausgebildet sind, ein Vernetzungselement (30) chemisch zu binden, welches ausgebildet ist, verunreinigende Zellen (20) auf ihrer Oberfläche chemisch zu binden und die Zellen von Interesse ungebunden zu halten (40), wobei die Membran ein erstes Volumen (V1) und ein zweites Volumen (V2) definiert, wobei
- das erste Volumen (V1) in Fluidverbindung mit einem Einlass steht, der ausgebildet ist, ein Probengemisch (M) in Kontakt mit der Membran zu injizieren, und das zweite Volumen (V2) in Fluidverbindung mit einem Auslass steht, der ausgebildet ist, die durch die Membran (10) eluierten Produkte zu sammeln,
- ein oder mehrere Pumpmittel, die ausgebildet sind, eine Druckdifferenz zwischen dem ersten Volumen (V1) und dem zweiten Volumen (V2) zu erzeugen, so dass der Druck in dem ersten Volumen (V1) höher ist als in dem zweiten Volumen (V2),
**dadurch gekennzeichnet, dass** die Filtrationsvorrichtung (50) ferner ein Mittel zum Umkehren der Druckdifferenz in einer Weise umfasst, dass der Druck in dem ersten Volumen (V1) niedriger wird als in dem zweiten Volumen (V2), und einen Rückgewinnungsauslass (54) in Fluidverbindung mit dem ersten Volumen (V1), aus dem die Zellen von Interesse zurückgewonnen werden können, oder einen spezifischen Einlass (53) auf der Seite der Membran umfasst, die ausgebildet ist, die Zellen von Interesse zu halten, und einen Auslass (54) umfasst, der auf derselben Seite der Membran wie der Einlass (53) angeordnet ist, aus dem die Zellen von Interesse (40) zurückgewonnen werden können.

12. Filtrationsvorrichtung (50) nach Anspruch 11, **dadurch gekennzeichnet, dass** sie ein Mittel zur Reduzierung des Totvolumens (55) umfasst, das es ermöglicht, das erste Volumen (V1) zu reduzieren.

13. Filtrationsvorrichtung nach Anspruch 11 oder 12, die ferner eine Schüttel- oder Vibrationsanordnung zum Schütteln oder Vibrieren der Membran (10) mit einer Frequenz von weniger als 10 000 Hertz umfasst.

14. Diagnostisches Verfahren zur Identifizierung und zum Nachweis von Krebs, Krebs in der Frühphase, genetischen Anomalien wie Down-Syndrom, **dadurch gekennzeichnet, dass** es ein Aufreinigungsverfahren nach den Ansprüchen 1 bis 10, unter Verwendung einer Filtrationsvorrichtung nach den Ansprüchen 11 bis 13 umfasst.

## Revendications

1. Procédé pour isoler des cellules d'intérêt (40) à partir d'un mélange échantillon (M) comprenant des cellules polluantes (20) comprenant un ou plusieurs marqueurs de surface connus (21), le procédé comprenant une étape de filtration (S1) à travers une membrane (10), dans lequel le mélange échantillon (M) passe à travers une membrane (10) en suivant un flux de filtration (F1), dans lequel la membrane (10) est pourvue de pores (11) et de liens (12), **caractérisé en ce que** le procédé comprend une étape d'ancrage (S3) permettant de lier les cellules polluantes (20) à la membrane (10) au moyen d'un élément d'ancrage (30) et de maintenir les cellules d'intérêt non liées (40) et une étape de récupération (S5), permettant de collecter les cellules d'intérêt (40), dans lequel ledit élément d'ancrage (30) comprend au moins un élément de liaison (31) capable de se lier spécifiquement audit un ou plusieurs marqueurs de surface (21) des cellules polluantes (20) ou d'une sous-population de cellules polluantes (20), et au moins un élément d'accroche (32) adapté pour coopérer avec les liens (12) de la membrane (10) et dans lequel l'étape de filtration (S1) fournit un mélange de déchets (M1) élué à travers la membrane (10) et un mélange récupéré (M2) restant dans la membrane (10), et **en ce que** la taille et la forme des pores (11) sont déterminées de manière à maintenir les cellules d'intérêt (40) dans le mélange récupéré (M2).

2. Procédé selon la revendication 1 , **caractérisé en ce que** les cellules d'intérêt (40) sont présentes dans ledit mélange échantillon (M) dans un rapport inférieur à 10⁻⁷, 10⁻⁸ ou 10⁻⁹, par rapport aux autres populations de cellules dans l'échantillon.

3. Procédé selon l'une quelconque des revendications précédentes, **caractérisé en ce que** l'étape d'ancrage (S3) comprend une étape (S3a) de mise en contact d'un élément d'ancrage (30) avec les cellules polluantes (20) et la membrane (10), et dans lequel l'étape d'ancrage (S3) comprend une étape d'incubation (S3b).

4. Procédé selon l'une des revendications 1 à 3, **caractérisé en ce que** l'élément d'ancrage (30) comprend en outre une extension (33) entre l'élément de liaison (31) et l'élément d'accroche (32).

5. Procédé selon l'une des revendications 1 à 4, **caractérisé en ce que** pendant ladite étape de récupération (S5), un flux (F2) d'un fluide d'élution et appliqué à travers la membrane (10) dans une direction opposée au flux de filtration (F1) ou un flux (F3) est appliqué le long de la membrane (10) sur sa face contenant les cellules d'intérêt (40).

6. PRocédé selon la revendication 5, l'étape de récupération (S5) comprenant une étape d'agitation contrôlée (S5a) facilitant la libération des cellules non liées de la membrane (10).

7. Procédé selon l'une quelconque des revendications précédentes, **caractérisé en ce que** les cellules d'intérêt (40) sont des cellules tumorales circulantes, des cellules foetales, ou des cellules infectieuses.

8. Procédé selon l'une quelconque des revendications précédentes, **caractérisé en ce qu'**il comprend en outre une étape de sélection de marqueur (SM), dans laquelle l'élément de liaison (31) de l'élément d'ancrage (30) est sélectionné pour se lier spécifiquement à un marqueur de surface (20), dont la concentration surfacique (PM20) est significativement plus élevée à la surface des cellules polluantes (20) ou d'une sous-population de cellules polluantes (20), par rapport à sa concentration surfacique (PM40) à la surface des cellules d'intérêt (40).

9. Procédé selon l'une des revendications 1 à 8, dans lequel ledit mélange échantillon (M) est choisi parmi les fluides corporels tels qu'un échantillon de sang, un fluide lymphatique, un échantillon d'urine, un échantillon de biopsie.

10. Procédé selon l'une des revendications 1 à 9, dans lequel la concentration du marqueur de surface (PM20) des cellules polluantes (20) ou d'une sous-population des cellules polluantes (20) est supérieure à la concentration (PM40) du même marqueur de surface sur les cellules d'intérêt (40) d'un facteur 2, ou 10 ou 100 ou 1000 ou plus, ou le marqueur de surface sélectionné des cellules polluantes (20) est absent des cellules d'intérêt (40).

11. Dispositif de filtration (50) comprenant :
- une membrane poreuse (10) telle que définie dans la revendication 1, comprenant des liens (12) sur sa surface capables de lier chimiquement un élément d'ancrage (30) capable de lier chimiquement des cellules polluantes (20) sur sa surface et de maintenir les cellules d'intérêt (40) non liées, ladite membrane définissant un premier volume (V1) et un second volume (V2), où
- le premier volume (V1) est en connexion fluidique avec une entrée adaptée pour injecter un mélange échantillon (M) en contact avec la membrane et le second volume (V2) est en connexion fluidique avec une sortie adaptée pour recueillir les produits élués à travers la membrane (10),
- un ou plusieurs moyens de pompage adaptés pour fournir une différence de pression entre le premier volume (V1) et le second volume (V2) de sorte que la pression est plus élevée dans le premier volume (V1) que dans le second volume (V2),
**caractérisé en ce que** le dispositif de filtration (50) comprend en outre un moyen pour inverser la différence de pression de manière à ce que la pression devienne plus faible dans le premier volume (V1) que dans le second volume (V2) et une sortie de récupération (54) en connexion fluidique avec le premier volume (V1) à partir duquel les cellules d'intérêt peuvent être récupérées, ou une entrée spécifique (53) sur le côté de la membrane adaptée pour maintenir les cellules d'intérêt et une sortie (54) disposée sur le même côté de la membrane que l'entrée (53) à partir de laquelle les cellules d'intérêt peuvent être récupérées.

12. Dispositif de filtration (50) selon la revendication 11, **caractérisé en ce qu'**il comprend un moyen de réduction du volume mort (55) permettant de réduire le premier volume (V1).

13. Dispositif de filtration selon les revendications 11 ou 12, comprenant en outre un dispositif d'agitation ou de vibration pour agiter ou faire vibrer la membrane (10) à une fréquence inférieure à 10 000 hertz.

14. Procédé de diagnostic pour identifier et détecter un cancer, un cancer en phase précoce, des anomalies génétiques telles que le syndrome de Down, **caractérisé en ce qu'**il comprend un procédé de purification tel que décrit dans les revendications 1 à 10, utilisant un dispositif de filtration selon les revendications 11 à 13.
